(19) Europäisches Patentamt
European Patent Office
Office européen des brevets

(11) Publication number: **0 549 006 A2**

# (12) EUROPEAN PATENT APPLICATION

(21) Application number: **92203415.2**

(22) Date of filing: **09.11.92**

(51) Int. Cl.5: **C07F 9/54**, A01N 57/34, C07C 211/62, A01N 33/12

(30) Priority: **27.11.91 CH 3471/92**

(43) Date of publication of application: **30.06.93 Bulletin 93/26**

(84) Designated Contracting States: **DE FR GB SE**

(71) Applicant: **FMC CORPORATION (UK) LIMITED
Tennax Road, Trafford Park
Manchester M17 1WT(GB)**

(72) Inventor: **Wehner, Wolfgang
Wetzbach 34
W-6144 Zwingenberg(DE)**
Inventor: **Grade, Reinhardt
Eisenlohrstrasse 35
W-7800 Freiburg(DE)**

(74) Representative: **W.P. Thompson & Co.
Coopers Building, Church Street
Liverpool L1 3AB (GB)**

## (54) Ammonium and phosphonium salts and their use as biocides.

(57) New compounds of formula (I)

$[R^1{}_3YR^2]^+{}_nX^{n-}$ (I)

are described in which Y stands for a phosphorus or nitrogen atom, n indicates a number from 1-4 and corresponds to the valence of the anion $X^{n-}$, the residues $R^1$ are the same or different, and in the case where Y = N, indicate $C_2$-$C_4$-alkyl or benzyl, and in the case where Y = P, have the meaning $C_3$-$C_4$ alkyl, phenyl or cyclohexyl, $R^2$ represents $C_{10}$-$C_{14}$ alkyl, and $X^{n-}$ indicates one of the anions $(a)^{1-}$, $(Hb)^{1-}$, $(b)^{2-}$, $(H_2c)^{1-}$, $(Hc)^{2-}$, $(c)^{3-}$, $(H_3d)^{1-}$, $(N_2d)^{2-}$, $(Hd)^{3-}$ or $(d)^{4-}$, whereby

$(a)^{1-}$ indicates nitrate, cyanate, rhodanide, periodate, perchlorate, tetraphenyl borate, salicylate, thiosalicylate, O,O-di-$C_3$-$C_8$ alkyl dithiophosphate, $C_1$-$C_8$-alkyl xanthogenate, $ICl_2^-$, $IClBr^-$, $IBr_2^-$, $Cl_3^-$, $Br_3^-$ or an N,N-dialkyl dithiocarbamate of formula

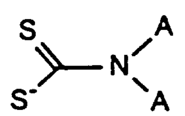

,

whereby each of the two residues A are $C_1$-$C_8$ alkyl or form the group $-NA_2$ together with a pyrrolidino, piperidino, or morpholino residue;

$(Hb)^{1-}$ is the hydrogen form of the anion $(b)^{2-}$, and $(b)^{2-}$ indicates sulfate, carbonate, ethylene-bis-dithiocarbamate of the formula

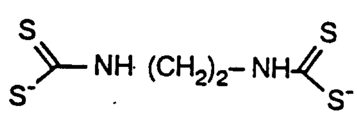

,

EP 0 549 006 A2

$[Fe(CN)_5(NO)]^{2-}$, $(Fe(SCN)_5(NO)]^{2-}$, or a complex anion $[Me(L)_4]^{2-}$,whereby Me stands for a zinc, copper or nickel atom, and L stands for cyanide or rhodanide;

$(H_2c)^{1-}$ and $(Hc)^{2-}$ are the dihydrogen and the hydrogen forms of the anion $(c)^{3-}$, and $(c)^{3-}$ indicates phosphate $[Cu(CN)_4]^{3-}$, $[Cu(SCN)_4]^{3-}$or a complex anion$[Me'(L)_6]^{3-}$, whereby Me' stands for an iron, cobalt, or manganese atom, and L stands for cyanide or rhodanide; and

$(H_3d)^{1-}$, $(H_2d)^{2-}$ and $(Hd)^{3-}$ are the trihydrogen, dihydrogen, and hydrogen forms of the anion $(d)^{4-}$, and $(d)^{4-}$ indicates a complex anion $[Me'(L)_6]^{4-}$, whereby Me' stands for an iron, cobalt, or manganese atom, and L stands for cyanide or rhodanide, with the prerequisite that in the case where Y = N, $X^{n-}$ is not N,N-dialkyl dithiocarbamate, tetraphenyl borate, nitrate, hydrogen sulfate, sulfate, phosphate, hydrogen or dihydrogen phosphate, and with the further provision that if Y = P and at the same time $X^{n-}$ = tetraphenyl borate, $R^1$ is not phenyl.

The compounds can be used as biocides with broad biological action spectrum.

The present invention concerns new ammonium and phosphonium salts, their use as biocides, a process for protecting technical materials and water systems from attack by harmful organisms and for combating such an attack, a well as combinations containing the compounds of the invention which are described below.

A number of publications, in part reaching far back in time, are concerned with quaternary ammonium compounds, e.g., pyridinium salts or compounds of the type $[(CH_3)NR_3]^+X$-, which have biocidal effects. The patents US-A-2,973,297 and US-A-2,812,332 are named as examples of this.

It is known from FR-A-1,058,618 how to introduce quaternary ammonium fluoborates such as dodecyldimethylbenzylammonium fluoborate onto textile fibers as an agent preventing decay.

A compound of the type $[N(C_2H_5)_2(CH_2C_6H_5)(C_{12}H_{25})]^+PF_6^-$ is known from JP-A-86-258,270 as a charge carrier for electrostatic image development.

GB-A-994,881 describes quaternary ammonium fluoborates, for example, N-alkyl dimethylbenzylammonium fluoborate as catalysts for the production of heat-hardening resins.

DE-A-2,530,522 instructs on the use of quaternary ammonium compounds, including dimethylbenzylalkyl-ammonium chloride, in disinfectants.

JP-A-89-121,262 describes quaternary ammonium salts of dimethyldithiocarbamic acid of the type:

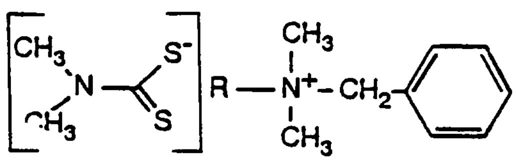

and its antimicrobial effect.

WO 89-10956 describes trimethyl-alkyl-ammonium dithiocarbamate, dimethylbenzyl-alkyl-ammonium dithiocarbamate and dimethyl-dialkylammonium dithiocarbamate; WO 88-02351 describes dimethylbenzylalkyl-ammonium salts and dimethyl-dialkyl-ammonium salts with the interhalogen anions $Br_3^-$ and $BrCl_2^-$.

A biocidal effect has also been reported for quaternary phosphonium compounds. Thus EP-A-332,578 instructs on the use of several tetraalkyl ammonium and tetraalkyl phosphonium salts, e.g., tri-n-butyl-n-tetradecylphosphonium tetrafluoborate as biocides.

US-A-4,874,526 describes the effectiveness of several tetraalkyl phosphonium bromides and chlorides, e.g., tri-n-butyl-n-tetradecylphosphoniumchloride as an antimicrobial agent for water treatment.

Surprisingly, new extremely effective biocides, with broad biological action spectrum based on quaternary ammonium and phosphonium as the cation, and specific inorganic, complex or organic anions, are provided by the present invention.

The present invention concerns compounds of the formula:

$[R^1_3YR^2]^+_nX^{n-}$ (I)

in which Y stands for a phosphorus or nitrogen atom, n indicates a number from 1-4 and corresponds to the valence of the anion $X^{n-}$, the residues $R^1$ are the same or different and in the case where Y = N, indicate $C_2$-$C_4$-alkyl or benzyl, and in the case where Y = P have the meaning $C_3$-$C_4$-alkyl, phenyl or cyclohexyl, $R^2$ represents $C_{10}$-$C_{14}$-alkyl, and $X^{n-}$ represents one of the anions $(a)^{1-}$, $(Hb)^{1-}$, $(b)^{2-}$, $(N_2c)^{1-}$, $(Hc)^{2-}$, $(c)^{3-}$, $(H_3d)^{1-}$, $(H_2d)^{2-}$, $(Hd)^{3-}$ or $(d)^{4-}$, whereby

$(a)^{1-}$ indicates nitrate, cyanate, rhodanide, periodate, perchlorate, tetraphenyl borate, salicylate, thiosalicylate, O,O-di-$C_3$-$C_8$ alkyl dithiophosphate, $C_1$-$C_8$ alkyl xanthogenate, $ICl_2^-$, $IClBr^-$, $IBr_2^-$, $Cl_3^-$, $Br_3^-$ or an N,N-dialkyl dithiocarbamate of the formula

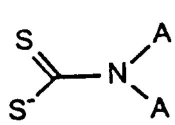 ,

whereby the two residues A are each $C_1$-$C_8$ alkyl or form the group $-NA_2$ together with a pyrrolidino, piperidino, or morpholino residue,

$(Hb)^{1-}$ is the hydrogen form of the anion $(b)^{2-}$ and $(b)^{2-}$ indicates sulfate, carbonate, ethylene-bis-dithiocarbamate of the formula

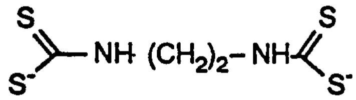 ,

$[Fe(CN)_5(NO)]^{2-}$,$[Fe(SCN)_5(NO)]^{2-}$ or a complex anion $[Me(L)_4]^{2-}$,whereby Me stands for a zinc, copper or nickel atom and L stands for cyanide or rhodanide,

$(H_2c)^{1-}$ and $(Hc)^{2-}$ are the dihydrogen and the hydrogen forms of the anion $(c)^{3-}$, and $(c)^{3-}$ indicates phosphate, $[Cu(CN)_4]^{3-}$, $[Cu(SCN)_4]^{3-}$ or a complex anion $[Me'(L)_6]^{3-}$, whereby Me' stands for an iron, cobalt, or manganese atom and L stands for cyanide or rhodanide, and

$(H_3d)^{1-}$, $(H_2d)^{2-}$ and $(Hd)^{3-}$ are the trihydrogen, dihydrogen, and hydrogen forms of the anion $(d)^{4-}$ and $(d)^{4-}$ indicates a complex anion $[Me'(L)_6]^{4-}$, whereby Me' stands for an iron, cobalt, or manganese atom, and L stands for cyanide or rhodanide; with the prerequisite that in the case where Y = N, $X^{n-}$ is not N,N-dialkyl dithiocarbamate, tetraphenyl borate, nitrate, hydrogen sulfate, sulfate, phosphate, hydrogen or dihydrogen phosphate, and with the further provision that if Y = P and at the same time $X^{n-}$ = tetraphenyl borate, $R^1$ is not phenyl.

$R^1$ in the meaning $C_2$ or $C_3$-$C_4$ alkyl includes, for example, ethyl, i-propyl, n-propyl, n-butyl, or i-butyl. $R^1$ preferably has the meaning n-butyl. The residues $R^1$ are preferably the same.

In compounds of formula I, $R^2$ is a straight-chain or branched $C_{10}$-$C_{14}$ alkyl residue and may represent, for example, n-decyl, n-undecyl, 1-methylundecyl, n-dodecyl, n-tridecyl or n-tetradecyl. $R^2$ preferably has the meaning n-tetradecyl. Isomeric mixtures of the given alkyl residues can also be appropriate.

The invention preferably concerns compounds of formula I, in which Y has the meaning of P.

The index n is particularly 1 or 2 and especially 1. Thus the mono-and divalent anions $X^{n-}$ named above are also preferred, particularly the monovalent one.

A is particularly $C_1$-$C_4$ alkyl. Of the alkyl xanthogenates, $C_1$-$C_4$ alkyl xanthogenates are preferred.

Compounds of formula I are appropriate in which n is 1 or 2 and $X^{n-}$indicates one of the anions hydrogen carbonate, hydrogen or dihydrogen phosphate, nitrate, rhodanide, periodate, perchlorate, sulfate, hydrogen sulfate, N,N-di-$C_1$-$C_4$ alkyl dithiocarbamate, O,O-di-$C_3$-$C_8$ alkyl dithiophosphate, salicylate, thiosalicylate, methyl xanthogenate, ethyl xanthogenate, $ICl_2^-$ or $IClBr^-$.

The compounds of formula I appropriately contain the same or different $C_3$-$C_4$ alkyl groups or phenyl as residues $R^1$, and $C_{12}$-$C_{14}$ alkyl as $R^2$. Those compounds are preferred in which residues $R^1$ are the same or different and indicate $C_3$-$C_4$ alkyl.

Compounds of formula I are preferred in which the cation corresponds to the formula $[(C_4H_9)_3P(C_{14}H_{29})]^+$.

Also preferred are compounds of formula I, in which $X^{n-}$ indicates nitrate, rhodanide, periodate, perchlorate, sulfate, hydrogen sulfate, N,N-di-$C_1$-$C_4$ alkyl dithiocarbamate, O,O-di-$C_3$-$C_8$ alkyl dithiophosphate, salicylate, thiosalicylate, ethyl xanthogenate, $ICl_2^-$ or $IClBr^-$.

Particularly preferred are compounds of formula I, in which $X^{n-}$indicates nitrate, rhodanide, hydrogen sulfate, N,N-dimethyldithiocarbamate or $ICl_2^-$.

Examples of compounds of formula I correspond to the formulas:

$[(n\text{-}C_4H_9)_3P(n\text{-}C_{14}H_{29})]^+$ $B(ph)_4^-$ (in which Ph stands for phenyl),
$[(n\text{-}C_4H_9)_3P(n\text{-}C_{14}H_{29})]^+$ $ICl_2^-$,
$[(n\text{-}C_4H_9)_3P(n\text{-}C_{14}H_{29})]^+$ $IClBr^-$,

$[(n\text{-}C_4H_9)_3P(n\text{-}C_{14}H_{29})]^+$ 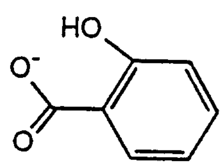 ,

$[(n\text{-}C_4H_9)_3P(n\text{-}C_{14}H_{29})]^+$ 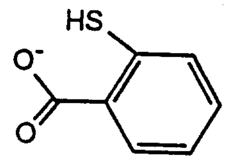 ,

$[(n\text{-}C_4H_9)_3P(n\text{-}C_{14}H_{29})]^+$ 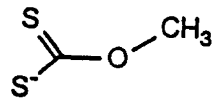 ,

$[(n\text{-}C_4H_9)_3P(n\text{-}C_{14}H_{29})]^+$ 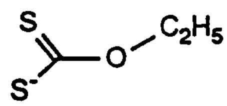 ,

$[(n\text{-}C_4H_9)_3P(n\text{-}C_{14}H_{29})]^+$ 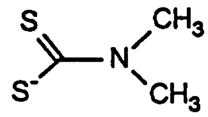 ,

$[(n\text{-}C_4H_9)_3P(n\text{-}C_{14}H_{29})]^+$ 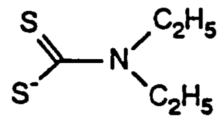 ,

$[(n\text{-}C_4H_9)_3P(n\text{-}C_{14}H_{29})]^+$ 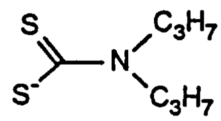 ,

$[(n\text{-}C_4H_9)_3P(n\text{-}C_{14}H_{29})]^+$ 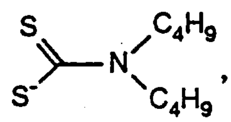 ,

$[(n\text{-}C_4H_9)_3P(n\text{-}C_{14}H_{29})]^+\ ClO_4$-,
$[(n\text{-}C_4H_9)_3P(n\text{-}C_{14}H_{29})]^+\ HSO_4^-$,
$[(n\text{-}C_4H_9)_3P(n\text{-}C_{14}H_{29})]_2^+\ SO_4^{2-}$,
$[(n\text{-}C_4H_9)_3P(n\text{-}C_{14}H_{29})]^+\ SCN^-$,
$[(n\text{-}C_4H_9)_3P(n\text{-}C_{14}H_{29})]^+\ OCN^-$,
$[(n\text{-}C_4H_9)_3P(n\text{-}C_{14}H_{29})]^+\ NO_3^-$,
$[(n\text{-}C_4H_9)_3P(n\text{-}C_{12}H_{25})]^+\ ClO_4^-$,
$[(n\text{-}C_4H_9)_3P(n\text{-}C_{12}H_{25})]^+\ HSO_4^-$,
$[(n\text{-}C_4H_9)_3P(n\text{-}C_{12}H_{25})]_2^+\ SO_4^{2-}$,
$[(n\text{-}C_4H_9)_3P(n\text{-}C_{12}H_{25})]^+\ SCN^-$,
$[(n\text{-}C_4H_9)_3P(n\text{-}C_{12}H_{25})]^+\ OCN^-$,
$[(n\text{-}C_4H_9)_3P(n\text{-}C_{12}H_{25})]^+\ NO_3^-$,
$[(i\text{-}C_3H_7)_3P(n\text{-}C_{14}H_{29})]^+\ ClO_4^-$,
$[(i\text{-}C_3H_7)_3P(n\text{-}C_{14}H_{29})]^+\ HSO_4^-$,
$[(i\text{-}C_3H_7)_3P(n\text{-}C_{14}H_{29})]_2^+\ SO_4^{2-}$,
$[(i\text{-}C_3H_7)_3P(n\text{-}C_{14}H_{29})]^+\ SCN^-$,
$[(i\text{-}C_3H_7)_3P(n\text{-}C_{14}H_{29})]^+\ OCN^-$,
$[(i\text{-}C_3H_7)_3P(n\text{-}C_{14}H_{29})]^+\ NO_3^-$,
$[(i\text{-}C_3H_7)_3P(n\text{-}C_{12}H_{25})]^+\ ClO_4^-$,
$[(i\text{-}C_3H_7)_3P(n\text{-}C_{12}H_{25})]^+\ HSO_4^-$,
$[(i\text{-}C_3H_7)_3P(n\text{-}C_{12}H_{25})]_2^+\ SO_4^{2-}$,
$[(i\text{-}C_3H_7)_3P(n\text{-}C_{12}H_{25})]^+\ SCN^-$,
$[(i\text{-}C_3H_7)_3P(n\text{-}C_{12}H_{25})]^+\ OCN^-$,
$[(i\text{-}C_3H_7)_3P(n\text{-}C_{12}H_{25})]^+\ NO^{3-}$,
$[(C_6H_5)_3P(n\text{-}C_{12}H_{25})]^+\ ClO_4^-$,
$[(C_6H_5)_3P(n\text{-}C_{12}H_{25})]^+\ HSO_4^-$,

$[(C_6H_5)_3P(n\text{-}C_{12}H_{25})]_2^+\ SO_4^{2-}$,
$[(C_6H_5)_3P(n\text{-}C_{12}H_{25})]^+\ SCN^-$,
$[(C_6H_5)_3P(n\text{-}C_{12}H_{25})]^+\ OCN^-$,
$[(C_6H_5)_3P(n\text{-}C_{12}H_{25})]^+\ NO_3^-$,
$[(C_6H_5)_3P(n\text{-}C_{10}H_{21})]^+\ ClO_4^-$,
$[(C_6H_5)_3P(n\text{-}C_{10}H_{21})]^+\ HSO_4^-$,
$[(C_6H_5)_3P(n\text{-}C_{10}H_{21})]_2^+\ SO_4^{2-}$,
$[(C_6H_5)_3P(n\text{-}C_{10}H_{21})]^+\ SCN^-$,
$[(C_6H_5)_3P(n\text{-}C_{10}H_{21})]^+\ OCN^-$,
$[(C_6H_5)_3P(n\text{-}C_{10}H_{21})]^+\ NO_3^-$.

In the formulas, the prefix n- indicates each time a straight-chain residue; $n\text{-}C_4H_9$ indicates, for example $-CH_2-CH_2-CH_2-CH_3$; $i\text{-}C_3H_7$ indicates isopropyl.

The compounds of the invention may be prepared in a way known in and of itself.

For this purpose, for example, the respective ammonium and phosphonium halides are reacted with salts, e.g., alkali metal salts, which contain the desired anions, to form the corresponding new ammonium and phosphonium salts of the invention.

For example, ammonium rhodanide or potassium rhodanide or ammonium tetraphenyl borate can be reacted with a quaternary or phosphonium halide $R^1{}_3YR^2Hal$ (Hal = halogen). The reaction conditions are not critical, and the reaction may be conducted, e.g., at normal pressure and at room temperature, and, for example, with wator as a solvent. Also, processing is conducted according to known methods; for example, the reaction products can be extracted and these can be obtained in pure form after distilling off the solvent. The initial materials are also known in and of themselves and in part represent commercial products.

The compounds of the invention are excellent biocides and have a broad biological action spectrum in various loci or substrates, so that they can be used generally, e.g., for protecting technical material. In particular, they are effective against both macro- and micro- organisms, e.g., bacteria, fungi and algae, protozoa, mollusks, mussels, barnacles, bryozoa, hydroids, etc.

The compounds of formula I are thus used as biocides according to the invention, particularly against the above named organisms, e.g., as technical biocides in material protection. The use also forms a subject of this invention. Another subject of the invention is a process for the protection of technical equipment or water systems from attack by damaging organisms and for combating harmful organisms in water systems, lubricants, hydraulic or metal working fluids, as well as in or on solid or porous technical materials, characterized by the fad that a compound of formula I is mixed with the given materials or systems or introduced as a solution onto their surface. Thus a process is of particular interest in which, for the technical materials or water systems to be protected, one is dealing with cold-water or process-water systems, wood, pulp, paper, plastics, paints, lacquers, lubricants, hydraulic or metal working fluids, drilling or cutting oils, petroleum, motor fuels, fats, waxes, leather, textiles, rubber, adhesives, or glasses.

It should be mentioned that the compounds of formula I may develop EP/AW properties upon application in lubricants, hydraulic or metal working fluids.

Correspondingly, the use of the compounds of the invention as biocides for technical materials and water systems is particularly emphasized. The compounds are thus particularly applicable as wood protection agents, as well as agents for the protection of pulp, paper, leather, textiles, rubber, plastics, paints, lacquers, lubricants, hydraulic and metal working fluids, drilling and cutting oils, petroleum, motor fuels, fats, waxes, adhesives and glasses. Also their applicability is underscored for the protection of wood, plastics, paints and lacquers and industrial water circuits, as well as also for the protection of wood and lacquer paints and antifouling coatings.

The compounds of formula (I) also find application as biocides in technical water systems, cold-water circuits and process-water systems, whereby they can also be applied to geothermal water, water in heating systems or air- conditioning plants or water for hydrostatic investigations.

One application of the invention encompasses also an application for preserving lubricants, hydraulic and metal working fluids as well as an application for the disinfecting of surfaces. The use of sufficiently water-soluble compounds is preferred for disinfectants and in industrial cleaning.

Appropriately, the compounds of the invention are suitable as preservatives for technical solutions used as additives for building materials, particularly for mortars, plasters (interior and exterior finish, floors, etc.) or for mixtures containing hydraulic binders, such as concrete, as additives for metal working fluids, particularly for drilling and cutting oils, as well as for rolling-mill, forging, separating and lubricating materials, as additives for coating substances, appropriately for paints and lacquers, preferably for latex

paints, as an active ingredient in coatings preventing or blocking decay, so-called antifouling paints, for furnishing biocides in surface coatings in general, and of wood, plastics, polymer materials, paper, leather, textiles in particular for surface treatment or for incorporating into building materials and building elements of polymers, as an antislime agent in water systems, preferably in systems for cold water and also in industrial water, particularly for the cellulose-processing industries, such as the paper industry, and finally for disinfection.

The present invention thus also encompasses combinations containing (a) a material to be protected against attack by damaging organisms, and (b) at least one compound of formula I.

In the case of material (a) this is, for example, a technical material or a water system. The technical material can thus be, e.g., wood, pulp, paper, plastics, paints, lacquers, lubricants, hydraulic or metal working fluids, drilling or cutting oils, petroleum, motor fuels, fats, waxes, leather, textiles, rubber, adhesives or glasses. In particular these are wood, plastics, paints, lacquers, particularly wood, objects of polyvinyl chloride or latex or antifouling paints.

For the material (a) to be protected, lubricants, hydraulic or metal working fluids are preferred. Also preferred are drilling or cutting oils which form component (a) of the combination of the invention.

In the case where component (a) of the combination of the invention is a water system, this is preferably cold water or process water.

The effect of the compounds which can be used according to the invention may advantageously be utilized in preservative and disinfecting finishes for plastics, e.g., polyamides, polyurethane foams, polyethylene, polypropylene, polybutylene, polyisobutylene, or polyvinyl chloride. When plasticizers are used, it is advantageous to add the antimicrobial additive to the plastic dissolved or dispersed in the plasticizer. The plastics with antimicrobial finishes may find application for objects of all types of use, in which an effectiveness against the most varied microorganisms is desired, such as against bacteria and fungi, thus, e.g., in soft PVC sheets, for floor mats, bathroom curtains, seating, stair grids in swimming pools, wall coverings, etc.

If paints (coating formulations) are protected, these are preferably latex paints and antifouling paints. For example, these contain lacquer raw materials known to the expert, such as binding agents, natural and synthetic resins, homopolymer and copolymer products with the monomers vinyl chloride, vinylidine chloride, styrene, vinyltoluene, vinyl esters, acrylic acids, and methacrylic acids, as well as their esters, and, in addition, chlorinated rubber, natural and synthetic rubber, chlorinated or cyclized as the case may be, also reaction resins such as epoxy resins, polyurethane resins, unsaturated polyesters, which can be converted into film-forming, higher molecular products, as the case may be, by addition of accelerators.

When used for protection of wood, in particular the outstanding effect of the compounds of formula I against the fungi and bacteria causing the rotting of wood is utilized. Appropriately, the wood to be protected is treated with a formulation (e.g., wood protection translucent film), which contains the compound of formula I. In this way, e.g., building materials of wood, or even buildings, which consist completely or partially of wood, can be protected. The compounds of formula I are very active in particular against blue and mold fungi in temporary wood protection, i.e., they are particularly suitable for treatment of fresh wood.

Particularly emphasized is the use of the compounds of formula I as biocides in water systems. The compounds are particularly suitable for preventing or reducing the slime formation in water systems, for example, in industrial water circuits, e.g., in cooling circuits.

In general the following water systems (among others) can be protected by means of the biocides of formula I:

Industrial cold water, e.g., for power plants or chemical plants, for steel and paper factories, as well as for breweries. These can thus be closed or open circuits, e.g., with cooling towers; also process wastewaters, particularly those which contain nutrients for microorganisms, such as, e.g., wastewater from paper factories and breweries; also injected water for oil fields or water which is utilized in the reverse osmosis process, e.g., in industrial application or for boiler water.

Other water systems in which the compounds of formula I may be used include circuit water in the paper industry, cooling water in fertilizer production, in oil refineries, in metal production (e.g., steel and copper), in the petrochemical and rubber-producing industry as well as in the textile and fiber industry, in gas production, in mineral processing, in glass, ceramics, food, and leather manufacturing, in heavy and light industries including metals and automobile production, in furniture manufacture, in the electronics industry, in the paint and adhesives industry and in other manufacturing operations.

Other fields of application concern the treatment of geothermal water, of water in central heating and air-conditioning plants in private, public, or industrial buildings, of water for hydrostatic investigations of pipelines and vessels, of water for swimming pools and of cooling water for marine engines, as well as of industrial pools.

In particular, in the combating of harmful organisms in water systems such as water circuits (e.g., in cold water systems), commonly used additional adjuvants may be added simultaneously with the use of the compounds of formula I of the invention, such as e.g., corrosion inhibitors, scaling inhibitors, agents for water softening, complexing agents, e.g., polymeric phosphites, phosphates, amides of phosphoric acid, phosphonic acids, polymeric carboxylic acids, of e.g., acrylic acid or maleic acid, their anhydrides or salts and other additives.

Of particular practical significance is the use of the compounds of formula I as biocides in industrial water circuits, in swimming pools and in industrial pools; in cooling and process water, particularly in cooling water in power plants, chemical plants, steel or paper factories, breweries, ships or marine engines; in injected water for oil fields; as well as in geothermal water or water in central heating systems, air-conditioning plants or in water for hydrostatic investigations.

A particular further field of application is the preservation of metal working fluids (MWF).

MWF are used in the processing of metals for cooling, lubricating, and rinsing (removal of chips).

There are different mineral, partially synthetic and fully synthetic MWF. In combating harmful organisms, other common additives may be added simultaneously with the use of the compounds of formula I according to the invention, e.g., such as emulsifiers, corrosion inhibitors, additives for improving lubricating properties, antifoaming agents, high pressure additives, (EP [extreme pressure] active materials) and other additives.

Another field of application of the present invention is the disinfection of surfaces. These can be of the most varied materials, e.g., of wood, plastic, metal, etc. In particular, surfaces of furniture and objects used in hospitals should be mentioned.

The quantity of compound of formula I, which is added to the water systems and the technical materials or introduced onto the latter, extensively depends, of course, on the respective type of application and may thus vary within wide limits. Due to the excellent effectiveness of the compounds of formula I, low concentrations are used when compared with analogous agents, whereby particularly favorable ecological aspects are also produced.

The compounds are utilized in concentration ranges known to the expert, depending on the application purpose. Whereas in water systems (cooling water, etc.), concentrations in the ppm range are sufficient, in antifouling recipes, concentrations of up to 40 wt.% are common.

Due to the broad spectrum of application, for example, with respect to the substrate or locus to be treated, concentrations of the compound of formula I of 0.1 ppm to 40 wt.% are considered. Typical concentrations in wood-protection agents are 0.01-10, particularly 0.1-5 wt.%; approximately the same as in the finishing of plastics, such as, e.g., soft PVC sheets; 0.1-40, particularly 0.5-20, e.g., 0.5-10 wt.% in paints, such as, e.g., latex and antifouling paints; 0.005-5, particularly 0.005-1 wt.% in petroleum, motor fuels, oils, fats, waxes, drilling and cutting oils, lubricants, etc.; as well as 0.01-1000, particularly 0.05-100, particularly 0.05-50, e.g., 1-40, but also 0.05-10 ppm in water systems, such as in cooling circuits, etc.

The compounds of formula I may be applied in pure form or together with support materials, such as, e.g, formulation adjuvants. They may also be suspended in liquid coating agents, among others, whereby wetting agents or emulsifying agents, if necessary, for the formation of uniform dispersions, can promote the uniform distribution of the effective ingredient. Other biocides may also be added.

In summary, let us introduce one more time several important fields of application for the compounds of formula I. These latter are suitable, e.g., as has already been mentioned, as preservatives for technical solutions, as additives to building materials, preferably for mortars, plasters (interior and exterior finishes, floors, etc.) or for hydraulic mixtures containing binders, such as concrete, as additives for metal working fluids, preferably for drilling and cutting oils, also for rolling-mill, forging, separating and lubricating materials, as an additive to coating materials, appropriately for paints and lacquers, preferably for latex paints as an active medium in coatings hindering or blocking decay, so-called antifouling coatings, for biocidal finishing of surface coatings in general and of wood, plastics, polymer materials, paper, leather and textiles, for surface treatment or incorporation in building materials and building elements of polymer material, such as anti-slime agents in water systems, preferably in systems for cooling water and in industrial water, preferably of the cellulose-processing industry, such as paper production, and finally for disinfection.

In addition to application in water systems, where in particular an extraordinarily good effect can be observed against the slime formation caused by bacteria, a further preferred field of application is in protective coatings, particularly in antifouling paints, which contain in addition to the usual basic substances and additives, e.g., 0.5-40 wt.%, preferably 3-15 wt.% of at least one compound of formula I with respect to the total mixture.

Usual basic substances for antifouling paints are lacquer raw materials denoted binders and known to the expert, such as natural and synthetic resins, homopolymer and copolymer products with the monomers vinyl chloride, vinylidine chloride, styrene, vinyltoluene, vinyl esters, vinyl alcohols, acrylic acid and methacrylic acid, as well as their esters, polyester and polyamide resins, also chlorinated rubber, natural and synthetic rubber, chlorinated or cyclized as the case may be, and also reaction resins, such as epoxy resins, polyurethane resins, unsaturated polyesters, which may be converted into film-forming higher molecular products, as the case may be, by addition of accelerators.

The binders may be liquid or may be present in dissolved form. In the case of dissolved binders, even thermoplastics, a protective film can also be formed by evaporating the solvent. Solid coating agents can be introduced onto objects, e.g., in powder coating processes. Other common base materials include, e.g., tar, modifiers, coloring materials, inorganic or organic pigments, fillers and accelerators.

The compounds of formula I may also finally find application in elastomer coatings, as well as in polymers containing silicone elastomers and fluorine.

In practice, the effective ingredients are used many times in combination with other biocides. In addition, the compounds of formula I may be combined with other biocides. In antifouling paints, combinations of products have often proven advantageous. Thus, the compounds of the invention may be combined e.g., with $Cu_2O$, CuSCN, zinc oxide, triorganotin compounds, such as tributyl tin fluoride or triphenyl tin fluoride, metallic copper or triazines or generally with those compounds which are known to the expert as effective against animal or plant growth.

Another form of application of the compounds of formula I is incorporation in plastics or natural or synthetic rubbers, or the introduction onto surfaces of shaped pieces made of these plastics, e.g., polyvinyl chlorides and their copolymers and mixed polymers, polyalkylenes, polyacrylates, polystyrenes, copolymers of these, polyurethanes or polyisocyanates, polyesters, epoxy resins, etc.

In particular, their use in plastics or polymer materials which find application as building materials is of importance, for example, for those which are subjected to weather conditions or are utilized in moist or wet regions. Thus, for example, roofing materials or paneling made of polyvinyl chloride, butyl rubber, chlorinated polyethylene, polyisobutylene, chloroprene, and chloroisoprene, EPDM as well as PVC mixed polymers with vinyl acetate or ethylvinyl acetate, polyacrylonitrile styrene, as the case may be, in mixture with fibrous fillers (if necessary also blended with bitumen), or foamed polyvinyl chlorides or polystyrenes can be mentioned as insulation materials protecting against heat and cold.

The compounds of formula I are suitable for the given applications as well as for other application. They are non-hygroscopic and heat stable and cover a wide application range with respect to their water solubility.

The invention also encompasses combinations (formulations) containing at least one compound of formula I. The form and type of the respective combination containing the compound of the invention is adapted to the application purpose.

For application, the compounds of formula I may be present in the following processing forms (in which the weight percentage data in parentheses represent advantageous quantities of effective ingredients):

Solid processing forms:

Dusting agents and powdering agents (up to 10%) granules, enveloping granules, impregnating granules, and homogeneous granules, pellets (grains) (1-80%).

Liquid processing forms:

a) Effective ingredient concentrates which can be dispersed in water:
Wettable powders and pastes (25-90% in commercial packaging, 0.01 - 15% in ready-to-use solutions), emulsion and solution concentrates (10-50%; 0.01-15% in ready-to-use solutions);
b) Organic solutions (0.1-20%); aerosols.

The invention also encompasses other agents which contain the compounds of the invention, as well as the use of the compounds of the invention and agents for combating harmful organisms (e.g., bacteria, fungi, algae, protozoa, mollusks, mussels, barnacles, bryozoa, hydroids, etc., particularly in material protection.

The biocidal agents of the invention may also contain other active substances or the compounds of formula I may be utilized together with other biocidal effective ingredients in the types of applications named above.

Examples of this are:

a) organosulfur compounds, e.g., methylene-bis-thiocyanate (MBT), isothiazolone or 3,5-dimethyltetrahydro-1,3,5-2H-thiodazine-2-thione (DMTT). Such substances are applied particularly against slime formation in papermaking.

b) Chlorinated phenols such as sodium pentachlorophenolate. Such compounds are characterized by a very broad action spectrum.

c) Copper salts, such as $Cu_2O$, CuSCN, copper sulfate, and copper nitrate, as additional algicides.

d) 2,2-dibromo-3-nitrilopropionamide (DBNPA) as an algicide, fungicide, and bactericide.

e) Chlorine and bromine as algicides and bactericides, which are particularly useful in water treatment.

f) Chlorine dioxide, chloroisocyanurate and hypochlorite as biocides, which may also be used particularly in water treatment.

g) Triazines, e.g., 2-methylthio-4-t-butylamino-6-cyclopropylamino-s-triazine, particularly as algicides.

h) Triorganotin compounds, e.g., bis-tributyltin oxide (TBTO), particularly as fungicides and algicides.

i) Wood biocides

ia) Salt mixtures based on silicofluorides, hydrogen fluorides, inorganic boron compounds, chromates, fluorides, arsenic (oxide, arsenate), copper salts (sulfate, naphthanate), tin and zinc salts, mercury compounds.

ib) Tar oil preparations

ic) Organic active ingredients, such as pentachlorophenol, phenol, DDT, dieldrin, lindane, gammexan [gammahexane?], chlorinated naphthalenes, dichlorofluanide, tributyltin compounds, pyrethoids, 3-iodo-2-propenyl-N-butyl carbamate, furmecyclox.

j) Disinfectants

ja) Phenol or phenol derivatives

jb) Formaldehyde and/or other aldehydes or derivatives

jc) Chlorine, organic or inorganic substances with active chlorine

jd) ampholytic surfactants

je) quaternary onium compounds.

Of course, other substances and adjuvants can also be contained in such formulations, as are usually used together in such preparations. These include, e.g., anionic, cationic, or non-ionic surface-active substances, electrolytes, complex formers, solubilizers, as well as coloring agents and fragrances. These additives, for example, serve for improving wetting capacity, hardening stability, for adjusting viscosity and for increasing the cold stability of solutions.

When used in latex paints and plasters, the compounds of formula I may also be combined with other fungicides. In water treatment, combinations with a bactericide are possible in order to combat slime-forming bacteria. Such combinations can bring about advantages with respect to technical application. In many cases, the combination with other algicides is also of advantage.

As has already been mentioned, the present invention also concerns a process for protecting technical materials and water systems from attack by harmful organisms or for combating these organisms by being placed on or in technical materials and water systems, and is hereby characterized in that a compound of formula I is incorporated with these materials or systems or is introduced onto them.

A process is preferred in which the technical materials or water systems include wood, pulp, paper, plastics, paints, lacquers, metal working fluids, drilling and cutting oils, petroleum, motor fuel, lubricants, fats and waxes, textiles, leathers, glasses, rubber and adhesives and industrial water circuits.

According to the invention, this is a process in which the compound of formula I is used in a quantity of 0.0001 to 40, particularly 0.01 to 10 wt.% with respect to the material to be protected.

The invention also encompasses a process in which a solvent containing 0.01 to 10, particularly 0.5 to 5 wt.% of the compound of formula I is utilized with respect to the weight of the solvent.

Of particular interest are processes in which another biocide is added.

Biocidal agents, which contain at least one of the above defined compounds of formula I in addition to the usual formulation components also represent a subject of the invention.

As has already been stated initially, the compounds of the invention have a very broad action spectrum against microorganisms. Thus, e.g., they are effective against pathogenic or slime-forming bacteria (gram-negative as well as gram-positive), algae and fungi, as well as e.g., mold fungi, blue fungi, and wood-disturbing fungi.

The following examples explain the invention in further detail, and in particular they show the good effectiveness of the compounds of formula I. The percents (%) given in these examples as well as in the remaining part of the description and in the Patent Claims indicate weight percent and parts by weight. The yields refer to the quantity of ammonium or phosphonium compound which is used.

**Example 1**

A solution of 55.9 g (0.574 mole) potassium rhodanide in 500 ml of water is added to a solution of 250 g (0.574 mole) of tri-n-butyl-n-tetradecylphosphonium chloride in 500 ml of water; the addition is made slowly at a temperature of 20-25 °C while stirring. The precipitated oil is taken up in 800 ml acetic ester while stirring, the organic phase is separated, and the aqueous phase is shaken up twice with 250 ml acetic ester. Finally, the organic phases are combined and washed twice, each time with 350 ml of water. After removing the solvent by application of partial pressure, 262.3 g (99.8% of the theoretical) are obtained of the product of formula $[(n\text{-}C_4H_9)_3P\ n\text{-}C_{14}H_{29}]^+SCN^-$ as a viscous oil;

| Elemental analysis: | | | | | |
|---|---|---|---|---|---|
| | C | H | N | P | S |
| theoretical:<br>experimental | 70.8%<br>70.6% | 12.3%<br>12.2% | 3.1%<br>3.1% | 6.8%<br>6.9% | 7.0%<br>6.7% |

The residual chloride content was determined to be less than 0.1%.

**Example 2**

214 g (0.468 mole) of the product from Example 1 are treated dropwise with stirring and ice cooling with 908 g (9.75 moles) concentrated $H_2SO_4$ in a 1-liter 3-necked flask equipped with a stirrer, thermometer, and reflux condenser with a connected gas washing bottle ($NaOH/H_2O$). The temperature should not exceed 40 °C; the reaction takes place with the evolution of COS. After the addition has been terminated, the material is heated to 75 °C for 3 hours while stirring. After cooling, the mixture is stirred into 1.3 liter of ice water, which is then extracted 3 times with 500 ml of dichloromethane each time. After distillation of the volatile components in vacuum, 473.3 g (87.9% of the theoretical) of the product of formula $[(n\text{-}C_4H_9)_3P\ n\text{-}C_{14}H_{29}]^+HSO_4^-$ remained as a highly viscous fluid optionally miscible with water.

| Elemental analysis: | | | | |
|---|---|---|---|---|
| | C | H | P | S |
| theoretical:<br>experimental: | 62.9%<br>63.0% | 11.6%<br>11.4% | 6.2%<br>6.2% | 6.5%<br>6.8% |

**Example 3**

43.5 g (0.1 mole) of tri-n-butyl-n-tetradecyl phosphonium chloride in 50 ml of water are added dropwise with stirring at a temperature of 20-25 °C to a 500-ml Erlenmeyer flask with a magnetic stirrer, which contains a solution of 10.2 g (0.12 mole) sodium nitrate in 100 ml of water. The precipitated oil is extracted twice, each time with 125 ml of dichloromethane. Then the organic phases are combined and washed twice with water. After removing the solvent by application of partial vacuum, 45.6 g (99.4% of the theoretical) of the product of formula $[(n\text{-}C_4H_9)_3P\ n\text{-}C_{14}H_{29}]^+NO_3^-$ of melting point 34 °C are obtained. The compound is difficultly soluble in water and readily soluble in alcohol. Dilutions of the alcohol solution with various quantities of water remain monophasic.

| Elemental analysis: | | | | |
|---|---|---|---|---|
| | C | H | N | P |
| theoretical:<br>experimental: | 67.6%<br>67.5% | 12.2%<br>11.8% | 3.0%<br>3.4% | 6.7%<br>6.8% |

**Example 4**

A solution of 8.3 g (0.025 mole) $K_3[Fe(CN)_6]$ in 50 ml of water is added dropwise while stirring to a solution of 30.3 g (0.07 mole) of ammonium chloride of formula $[(CH_3)_2N(CH_2C_6H_5)\ n\text{-}C_{14}H_{29}]^+Cl_3^-$x $2H_2O$ in 100 ml of water. The precipitate that is formed is suction filtered, washed free of chloride and dried. 29.3 g (97% of the theoretical) of the product of formula $[(CH_3)_2N(CH_2C_6H_5)\ n\text{-}C_{14}H_{29}]_3{}^+[Fe(CN)_6]^{3-}$ of melting point 210°C are obtained.

| Elemental analysis: | | | | |
|---|---|---|---|---|
| | C | H | N | Fe |
| theoretical:<br>experimental: | 74.5%<br>73.1% | 10.5%<br>10.9% | 10.4%<br>10.3% | 4.6%<br>4.6% |

**Example 5**

The compound $[(CH_3)_2N(CH_2C_6H_5)\ n\text{-}C_{14}H_{29}]^+Cl^-$ x $2H_2O$ is reacted with a corresponding quantity of potassium diisopropyldithiophosphate according to the procedure given in Example 4. The product, which is difficultly soluble in water, $[(CH_3)_2N(CH_2C_6H_5)\ n\text{-}C_{14}H_{29}]^+[S_2P(O^iC_3H_7)_2]^-$ of melting point 100°C is obtained in a yield of 91.4% of the theoretical:

| Elemental analysis: | | | | | |
|---|---|---|---|---|---|
| | C | H | N | P | S |
| theoretical:<br>experimental: | 63.8%<br>63.8% | 10.3%<br>10.4% | 2.6%<br>2.5% | 5.7%<br>5.6% | 11.7%<br>11.5% |

The residual content of chloride was determined to be less than 0.3%.

**Example 6**

The compound $[n\text{-}C_4H_9)_3\ n\text{-}C_{14}H_{29}]^+Cl^-$ is reacted with a corresponding quantity of sodium tetraphenyl borate according to the procedure given in Example 4 and is processed. The product which is difficultly soluble in water (solubility ≦ 20 ppm) $([n\text{-}C_4H_9)_3P\ n\text{-}C_{14}H_{29}]^+[B(C_6H_5)_4]^-$ of melting point 128°C is obtained in a yield of 97% of the theoretical:

| Elemental analysis: | | | | |
|---|---|---|---|---|
| | C | H | B | P |
| theoretical:<br>experimental: | 83.5%<br>83.6% | 10.7%<br>11.1% | 1.5%<br>1.1% | 4.3%<br>4.3% |

**Example 7**

The compound $[(n\text{-}C_4H_9)_3P\ n\text{-}C_{14}H_{29}]^+Cl^-$ is reacted with a corresponding quantity of sodium periodate according to the procedure given in Example 3. The product of formula $[(n\text{-}C_4H_9)_3P\ n\text{-}C_{14}H_{29}]^+[IO_4]^-$ which is difficultly soluble in water is obtained as a viscous oil in a yield of 96% of the theoretical.

| Elemental analysis: | | | | |
|---|---|---|---|---|
| | C | H | I | P |
| theoretical:<br>experimental: | 52.9%<br>53.8% | 9.6%<br>9.8% | 21.6%<br>20.6% | 5.2%<br>5.2% |

**Examples 8 and 9**

A solution of 43.5 g (0.1 mole) tri-n-butyl-n-tetradecylphosphonium chloride in 94 ml of water is dropped into a 500-ml 3-necked flask into a solution of 0.1 mole (16.2 g) iodomonochloride (= Example 8) or 0.1 mole (20.7 g) of iodomonobromide (= Example 9) in 200 ml of dichloromethane while stirring, such that the internal temperature of 25°C is not exceeded. The mixture is then stirred for 1 hour at 20-25°C, then the organic phase is separated and concentrated to a residue by application of partial pressure. Example 8 gives quantitatively the product of formula $[(n\text{-}C_4H_9)_3P\ n\text{-}C_{14}H_{29}]^+[ICl_2]^-$ as a brownish-red oil;

| Elemental analysis: | | | | | |
|---|---|---|---|---|---|
| | C | H | Cl | I | P |
| theoretical:<br>experimental: | 52.3%<br>52.8% | 9.4%<br>9.7% | 11.9%<br>11.2% | 21.2%<br>20.9% | 5.2%<br>5.3% |

Example 9 gives quantitatively the product of formula $[(n\text{-}C_4H_9)_3P\ n\text{-}C_{14}H_{29}]^+(IClBr)^-$ as a reddish-brown oil:

| Elemental analysis: | | | | | | |
|---|---|---|---|---|---|---|
| | C | H | Br | Cl | I | P |
| theoretical:<br>experimental: | 48.8%<br>48.9% | 8.8%<br>8.6% | 12.5%<br>12.6% | 5.5%<br>5.2% | 19.8%<br>19.7 | 4.8%<br>4.7% |

**Example 10**

An aqueous solution of 21.7 g (0.05 mole) tri-n-butyl-n-tetradecylphosphoniumchloride is reacted with 8.8 g (0.055 mole) sodium salicylate according to the method given in Example 3. The product of formula

$[(n\text{-}C_4H_9)_3P\ n\text{-}C_{14}H_{29}]^+$ 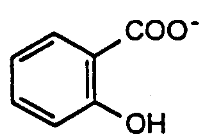

is obtained as a viscous oil in a yield of 99.3% of the theoretical.

| Elemental analysis: | | | |
|---|---|---|---|
| | C | H | P |
| theoretical (monohydrate):<br>experimental: | 71.3%<br>71.4% | 11.4%<br>11.4% | 5.5%<br>5.6% |

**Example 11**

A solution of sodium thiosalicylate is prepared from 7.71 g (0.05 mole) thiosalicylic acid, 2.0 g (0.05 mole) of NaOH and 80 ml of water. 43.5 g of a 50% aqueous solution of tri-n-butyn-tetradecyphosphonium chloride (0.05 mole) is dropped into this at a temperature of 20-25 °C while stirring. The precipitated oil is extracted in portions with a total of 250 ml of acetic ester. The organic phase is then washed twice with water and the volatile components are removed in vacuum. 27.7 g (= 93% of the theoretical) of the product of formula

$[(n\text{-}C_4H_9)_3P\ n\text{-}C_{14}H_{29}]^+$ 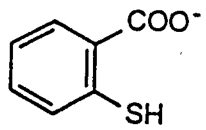

are obtained as a viscous oil.

| Elemental analysis: | | | | |
|---|---|---|---|---|
| | C | H | P | S |
| theoretical:<br>experimental: | 71.7%<br>71.6% | 11.1%<br>11.1% | 5.6%<br>5.7% | 5.8%<br>5.1% |

**Examples 12-16**

The description of the preparation of compounds 12-16 is tabulated in Table 1. Compounds 12-16 are prepared by reacting tri-n-butyl-n-tetradecylphosphonium chloride with the salts given in the table. The preparation is conducted essentially by the method described in Example 3, but acetic ester is used for the extraction. Tri-n-butyl-n-tetradecyl phosphonium chloride is utilized each time as a 50% aqueous solution.

**Example 17: Determination of the bacterial minimal inhibiting concentration**

The cultures of various bacterial strains cultured in Caso Peptone Bouillon for 24 hours at 30 °C are diluted in physiological NaCl solution 1:1000. Enough Caso Peptone Bouillon is added to the suspension each time so that the bacteria are diluted again to 1:1000. Subsequently the compounds given in Table 2 are added in quantities of 10, 30, 60 and 100 mg/l. Cations of the tested compounds are $[(n\text{-}C_4H_9)_3P\ n\text{-}C_{14}H_{29}]^+$ (= type P) or $[(CH_3)_2N(CH_2C_6H_5)(n\text{-}C_{14}H_{29})]^+$ (= type N). After culturing for 24 hours at 30 °C in an agitated water bath, the turbidity is evaluated. The minimal inhibiting concentration (MIC) is the concentration at which the bouillon is not turbid due to the bacterial growth. The following bacterial strains are utilized:

Strain

A: Bacillus subtilis
B: Serratia marcescens
C: Enterobacter aerogenes
D: Alcaligenes dentrificans
E: Proteus vulgaris
F: Pseudomonas fluorescens

**Example 18: Determination of the minimal concentration for killing a bacterial culture (MKC mixed culture)**

The "minimal killing concentration" (= MKC) corresponds to the concentration of an active substance, which is directly sufficient to kill the bacteria in buffer solution. Strains are used which are obtained from overnight cultures (one colony of each on Caso Peptone agar taken from a 24 hour culture, overnight culture (ONC).

The strains used are:

Escherichia coli
Bacillus cereus var. mycoides
Staphylococcus aureus
Enterobacter aerogenes
Proteus vulgaris
Pseudomonas aeruginosa.

0.5 ml of the ONC of each of the given strains are well shaken up together in an Erlenmeyer flask. The mixed culture thus obtained is added to a Tyrode buffer solution in a quantity such that a dilution of 1:1000 is obtained. Then the substance to be tested is added in the desired test concentration and cultured for 24 hours at 30 °C. After dripping 5 µl of the thus-treated cultures onto Caso Peptone plates, and a 24-hour culturing at 30 °C, the evaluation of the plates for growth is made as follows, with respect to the killing of the bacteria:

- = no growth, all bacteria killed
(-) = 1-10 colonies, bacteria killed for the most part
(+) = > 10 colonies, growth weaker than control, slight killing
\+ = growth as in control, no killing

**Example 19. Determination of the minimal inhibiting concentration (MIC) for fungi**

The investigation is undertaken with the known agar incorporation test in malt extract. 100 mg/l of the compound were added each time to the agar for inhibition. Incubation was conducted for 7 days at 28 °C. Table 4 shows the results of the test; + indicates growth (MIC > 100 mg/l), - indicates inhibition of the growth (MIC < 100 mg/l).

**Example 20. Determination of the effect against Chlorella vulgaris**

The culture grown in algae medium over a period of 14 days is diluted 1:200 in the algae medium. Then the compounds given in Table 5 are added, so that a concentration of 10 mg/l is obtained. After 6 hours of incubation at room temperature, 10 µl samples are taken and used for determination of the killing effect by dropping onto algae agar. After a culture of 14 days at room temperature, upon exposure to light, the growth in the algae medium is determined visually (determination of growth inhibition) and on algae agar (determination of the killing effect).

**Table 1: Preparation of tri-n-butyl-tetradecyl-phosphonium salts 12-16 from the corresponding chloride (PCL)**

| Example No. | Initial substance | Product | Yield (% of the theoretical) | Characterization;elemental analysis |
|---|---|---|---|---|
| 12 | 43.5 g (0.1 mole) PCL + 16 g potassium ethyl xanthogenate | 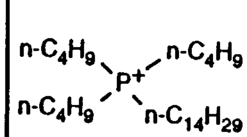 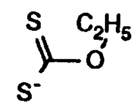 | 96% | viscous oil; theoretical:66.9%C, 11.8% H, 5.9% P, 12.3% S experimental: 66.8% C, 11.9% H, 6.1% P, 10.9% S |
| 13 | 43.5 g PCL + 14.3 g Na-dimethyldithiocarbamate (0.1 mole as aqueous solution) | 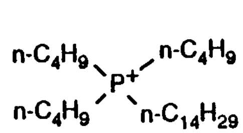 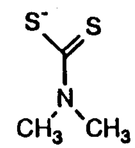 | 95% | viscous oil; residual Cl < 0.3% theoretical: 66.1%C, 12.2% H, 2.5% N, 5.9% P, 11.6% S, experimental: 66.3% C, 12.0% H, 2.7% N, 5.9% P, 12.2% S calculated with 0.3 mole $H_2O$ |
| 14 | 43.5 g (0.1 mole) PCL + 25.2 g K-diisopropyl-dithiophosphate | 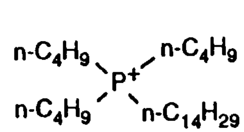 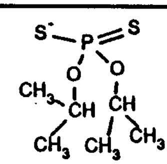 | 99.5% | viscous oil; residual Cl < 0.1% theoretical: 62.7% C, 11.5% H, 10.1% P, 10.5% S experimental: 62.6% C, 11.5% H, 10.0% P, 10.3%S |
| 15 | 43.5 g (0.1 mole) PCL + 27.3 g (0.12 mole) Na-dibutyldithiocarbamate (as an aqueous solution) | 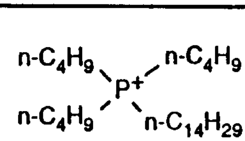 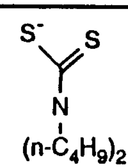 | quantitative | viscous oil; residual Cl < 0.3% theoretical: 69.1% C, 12.3% H, 2.3% N, 5.1% P, 10.5% S experimental: 68.9% c, 12.2% H, 2.4% N, 4.9% P, 10.7% S |
| 16 | 43.5 g (0.1 mole) PCL + 44.6 g (0.12 mole) ammonium dioctyl-dithiophosphate | 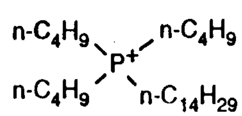 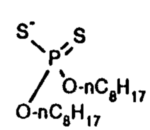 | 92.2% | viscous oil; residual Cl <0.1% theoretical: 66.2% C, 12.0% H, 8.1% P, 8.4% S experimental: 65.6% C, 11.9% H, 8.1% P, 8.9% S calculated with 0.5 mole $H_2O$ |

Table 2. Minimal inhibiting concentration (mg of the compound/l) against bacteria in liquid cultures; cations are $[(n\text{-}C_4H_9)_3P\ n\text{-}C_{14}H_{29}]^+$ (= type P) or $[(CH_3)_2N(CH_2C_6H_5)\ (n\text{-}C_{14}H_{29}]^+$ (= type N).

| compound of (mg/l) example no. | anion | cation type | minimal inhibiting concentration A | B | C | D | E | F |
|---|---|---|---|---|---|---|---|---|
| 1 | $SCN^-$ | P | 10 | 30 | 10 | 10 | 10 | 30 |
| 2 | $HSO_4^-$ | P | 10 | 30 | 10 | 10 | 10 | 30 |
| 3 | $NO_3^-$ | P | 10 | 30 | 10 | 10 | 10 | 30 |
| 4 | $Fe(CN)_6^-$ | N | <100 | <100 | <100 | <100 | <100 | <100 |
| 5 | $(iC_3H_7O-)_2PSS^-$ | N | <100 | <100 | <100 | <100 | <100 | <100 |
| 7 | $IO_4^-$ | P | 10 | 30 | 30 | 10 | 10 | 30 |
| 8 | $ICl_2^-$ | P | 10 | 30 | 30 | 10 | 10 | 30 |
| 9 | $IClBr^-$ | P | 10 | 30 | 30 | 10 | 10 | 30 |
| 10 | 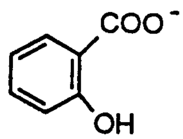 | P | 10 | 30 | 30 | 10 | 10 | 30 |
| 11 | 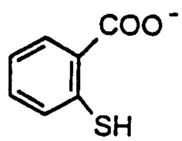 | P | 10 | 30 | 30 | 10 | 10 | 30 |
| 12 | $S_2COC_2H_5^-$ | P | 10 | 30 | 30 | 10 | 10 | 30 |
| 13 | $(CH_3)_2NCSS^-$ | P | 10 | 30 | 10 | 10 | 10 | 30 |
| 14 | $S_2P(OiC_3H_7)_2^-$ | P | 10 | 30 | 30 | 10 | 10 | 30 |

Table 3

| Minimal killing concentration (mg of compound/l) against a bacterial mixed culture after 24 hours in Tyrode solution | | | | |
|---|---|---|---|---|
| Compound of example no. | Effect after 24 h | | | |
| | 10 | 30 | 60 | 100 mg/l |
| 1 | - | - | - | - |
| 2 | - | - | - | |
| 3 | (-) | (-) | - | - |
| 5 | | (-) | - | - |
| 7 | (-) | - | - | - |
| 8 | - | - | - | |
| 9 | - | - | - | |
| 10 | - | - | - | - |
| 11 | - | - | - | - |
| 12 | - | - | - | |
| 13 | - | - | - | |
| 14 | - | - | - | |

Table 4

| Minimal inhibiting concentration against various fungi in the agar incorporation test, tested concentration of 100 mg/l | | | |
|---|---|---|---|
| Compound of example no. | Strain: | | |
| | Coriolus versicolor | Penicillium funiculosum | Aureobasidium pullulans |
| 1 | - | - | - |
| 2 | - | - | - |
| 3 | | - | - |
| 4 | - | - | - |
| 5 | - | - | - |
| 7 | | - | - |
| 8 | - | - | - |
| 9 | - | - | - |
| 10 | - | - | - |
| 11 | - | - | - |
| 12 | - | - | - |
| 13 | - | - | - |
| 14 | - | - | - |

Table 5

| Killing (after 6 hours) and inhibition of Chlorella vulgaris in algae medium, test concentration of 10 mg/l. | | |
|---|---|---|
| Compound from example no. | killing | inhibition |
| 1 | - | - |
| 2 | - | - |
| 3 | - | - |
| 5 | - | - |
| 7 | - | - |
| 8 | - | - |
| 9 | - | - |
| 10 | - | - |
| 11 | - | - |
| 12 | - | - |
| 13 | - | - |
| 14 | - | - |
| - = no further growth, algae killed, inhibited with respect to growth<br>+ = growth as in control, no killing. | | |

## Claims

**1.** Compounds of formula I

$[R^1_3YR^2]^+_nX^{n-}$ (I)

in which Y stands for a phosphorus or nitrogen atom, n is a number from 1 - 4 and corresponds to the valence of the anion $X^{n-}$,the residues $R^1$ are the same or different, and in the case where Y = N, indicate $C_2$-$C_4$-alkyl or benzyl, and in the case where Y = P, have the meaning $C_3$-$C_4$ alkyl, phenyl or cyclohexyl; $R^2$ represents $C_{10}$-$C_{14}$ alkyl, and $X^{n-}$ indicates one of the anions $(a)^{1-}$, $(Hb)^{1-}$, $(b)^{2-}$, $(H_2c)^{1-}$, $(Hc)^{2-}$, $(c)^{3-}$, $(H_3d)^{1-}$, $(H_2d)^{2-}$, $(Hd)^{3-}$ or $(d)^{4-}$, whereby

$(a)^{1-}$ indicates nitrate, cyanate, rhodanide, periodate, perchlorate, tetraphenyl borate, salicylate, thiosalicylate, O,O-di-$C_3$-$C_8$-alkyl dithiophosphate, $C_1$ -$C_8$-alkyl xanthogenate, $ICl_2^-$, $IClBr^-$, $IBr_2^-$, $Cl_3^-$, $Br_3^-$ or an N,N-dialkyl-dithiocarbamate of formula

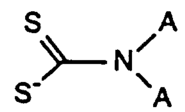 ,

whereby each of the two residues A are $C_1$-$C_8$-alkyl or form the group $-NA_2$ together with a pyrrolidino, piperidino, or morpholino residue;

$(Hb)^{1-}$ is the hydrogen form of the anion $(b)^{2-}$; and $(b)^{2-}$ indicates sulfate, carbonate, ethylene-bis-dithiocarbamate of the formula

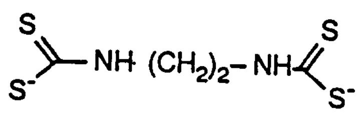 ,

$[Fe(CN)_5(NO)]^{2-}$, $[Fe(SCN)_5(NO)]^{2-}$, or a complex anion $[Me(L)_4]^{2-}$,whereby Me stands for a zinc, copper or nickel atom and L stands for cyanide or rhodanide;

$(H_2c)^{1-}$ and $(Hc)^{2-}$ are the dihydrogen and the hydrogen forms of the anion $(c)^{3-}$, and $(c)^{3-}$ indicates phosphate, $[Cu(CN)_4]^{3-}$, $[Cu(SCN)_4]^{3-}$ or a complex anion $[Me'(L)_6]^{3-}$, whereby Me' stands

for an iron, cobalt, or manganese atom and L stands for cyanide or rhodanide, and

$(H_3d)^{1-}$, $(H_2d)^{2-}$ and $(Hd)^{3-}$ are the trihydrogen, dihydrogen, and hydrogen forms of the anion $(d)^{4-}$ and $(d)^{4-}$, indicates a complex anion $[Me'(L)_6]^{4-}$, whereby Me' stands for an iron, cobalt, or manganese atom, and L stands for cyanide or rhodanide; with the prerequisite that in the case where Y = N, $X^{n-}$ is not N,N-dialkyl dithiocarbamate, tetraphenyl borate, nitrate, hydrogen sulfate, sulfate, phosphate, hydrogen or dihydrogen phosphate, and with the further provision that if Y = P and at the same time $X^{n-}$ = tetraphenyl borate, $R^1$ is not phenyl.

**2.** Compounds according to Claim 1, in which Y indicates phosphorus.

**3.** Compounds according to Claim 1, in which n is 1 or 2 and $X^{n-}$ indicates one of the anions hydrogen carbonate, hydrogen phosphate or dihydrogen phosphate, nitrate, rhodanide, periodate, perchlorate, sulfate, hydrogen sulfate, N,N-di-$C_1$-$C_4$-alkyl dithiocarbamate, O,O-di-$C_3$-$C_8$- alkyl dithiophosphate, salicylate, thiosalicylate, methyl xanthogenate, ethyl xanthogenate, $ICl_2^-$ or $IClBr^-$.

**4.** Compounds according to Claim 1, in which the residues $R^1$ are the same or different and indicate $C_3$-$C_4$- alkyl or phenyl and $R_2$ indicates $C_{12}$-$C_{14}$-alkyl.

**5.** Compounds according to Claim 1, in which the residues $R^1$ are the same or different and indicate $C_3$-$C_4$- alkyl.

**6.** Compounds according to Claim 2, in which the cation corresponds to the formula $[(C_4H_9)]_3P(C_{14}H_{29})]^+$.

**7.** Compounds according to Claim 2, in which $X^{n-}$ indicates nitrate, rhodanide, periodate, perchlorate, sulfate, hydrogen sulfate, N,N-di-$C_1$-$C_4$-alkyl dithiocarbamate, O,O-di-$C_3$-$C_8$-alkyl dithiophosphate, salicylate, thiosalicylate, ethyl xanthogenate, $ICl_2^-$ or $IClBr^-$.

**8.** Compounds according to Claim 2, in which $X^{n-}$ indicates nitrate, rhodanide, hydrogen sulfate, N,N-dimethyl dithiocarbamate or $ICl_2^-$.

**9.** Combinations containing
a) a material to be protected against attack by harmful organisms, and
b) at least one compound of formula I according to Claim 1.

**10.** Combination according to Claim 9, in which the material (a) is a technical material or a water system.

**11.** Combination according to Claim 10, in which the technical material is wood, pulp, paper, plastics, paints, lacquers, lubricants, hydraulic or metal working fluids, drilling or cutting oils, petroleum, motor fuels, fats, waxes, leather, textiles, rubber, adhesives or glasses.

**12.** Combination according to Claim 9, in which the material is wood, objects made of polyvinyl chloride or latex paints or antifouling paints.

**13.** Combination according to Claim 9, in which material (a) is a lubricant, hydraulic or metal working fluid.

**14.** Combination according to Claim 9, in which material (a) is a drilling or cutting oil.

**15.** Combination according to Claim 10, in which cooling water or process water represents the water system.

**16.** A method of controlling organisms in substrates or loci selected from technical materials and water systems, comprising contacting the organisms, loci or substrates with an effective amount of a compound of formula I of claim 1.

**17.** A method according to Claim 16 wherein the loci or substrates are selected from wood, pulp, paper, leather, textiles, rubber, plastics, paints, lacquers, lubricants, hydraulic and metal working fluids, drilling and cutting oils, petroleum, motor fuels, fats, waxes, adhesives and glasses.

**18.** A method according to Claim 16 wherein the loci or substrates are selected from wood, plastics, paints and lacquers and industrial water circuits.

**19.** A method according to Claim 16 wherein the loci or substrates are selected from water systems, cooling water circuits and process water systems.

**20.** A method according to Claim 19, in which the water system is geothermal water, water in heating systems or air- conditioning plants or water for hydrostatic investigations.

**21.** Process for protecting technical materials or water systems from attack by damaging organisms and for combating harmful organisms in water systems, lubricants, hydraulic or metal working fluids as well as on or in solid or porous technical materials, characterized in that a compound of formula I according to Claim 1 is mixed with the given materials or systems or is introduced as a solution onto their surface.

**22.** Process according to Claim 21, in which the technical materials or water systems to be protected are selected from cooling water or process-water systems, wood, pulp, paper, plastics, paints, lacquers, lubricants, hydraulic or metal working fluids, drilling or cutting oils, petroleum, motor fuels, fats, waxes, leather, textiles, rubber, adhesives and glasses.

**23.** Process according to Claim 21, in which the compound of formula I is utilized in a quantity of 0.0001 to 40, particularly 0.01 to 10 wt.% with respect to the material to be protected.

**24.** Process according to Claim 21, in which a solution containing 0.01-10, particularly 0.5-5 wt.% with respect to the weight of the solvent, of the compound of formula I is utilized.